# EUROPEAN PATENT SPECIFICATION

(11) **EP 3 482 781 B1**
(45) Date of publication and mention of the grant of the patent: **30.11.2022**
(21) Application number: 18159540.6
(22) Date of filing: 01.03.2018
(51) Int. Cl.: A61L 27/56, A61L 27/58

(54) **METHOD FOR PREPARING FIBROUS SCAFFOLD FOR PERSONALIZED TISSUE ENGINEERING**
VERFAHREN ZUR HERSTELLUNG EINES FASERGERÜSTS FÜR PERSONALISIERTE GEWEBEZÜCHTUNG
PROCÉDÉ DE PRÉPARATION D'UN ÉCHAFAUDAGE FIBREUX POUR INGÉNIERIE DE TISSUS PERSONNALISÉE

(30) Priority: 09.11.2017 KR 20170148825
(43) Date of publication of application: 15.05.2019
(73) Proprietor: Dankook University Cheonan Campus Industry Academic Corporation Foundation, Cheonan-si, Chungcheongnam-do 31116 (KR)
(72) Inventor: KIM, Hae-Won, 31170 Cheonan-si, Chungcheongnam-do (KR); LEE, Jung Hwan, 31116 Cheonan-si, Chungcheongnam-do (KR); LEE, Hae-Hyoung, 31179 Cheonan-si, Chungcheongnam-do (KR); MANDAKHBAYAR, Nandin, 31116 Cheonan-si, Chungcheongnam-do (KR); CAMPBELL KNOWLES, Jonathan, St Albans, Hertfordshire AL3 5AJ (GB)
(74) Representative: Regimbeau

(56) References cited:
- KR-A- 20160 073 811
- US-A1- 2011 250 308
- US-A1- 2014 234 457

## Description

### [Technical Field]

The present invention relates to a method for preparing a cotton-like fibrous scaffold which can be suitably used for defects in various forms and has a three-dimensional (3D) volume; a scaffold prepared by the method; and use of the scaffold.

### [Background Art]

The advent of an aging society and increase of patients having bone defects have resulted in recent research endeavoring to induce bone regeneration. In particular, non-absorbable biomaterials, such as metals and ceramics, are already widely used, but have difficulty in direct substitution of localized bone defects, and there is the occasional risk and inconvenience of secondary surgery for removal after recovery. Under the circumstances, studies on procedures with artificial bone have emerged using biodegradable polymers which induce *in vivo* regeneration of defective bone, and by degrading with the passage of time, require no additional procedure for removal. State-of-the-art tissue engineering increasingly involves preparation of tissue-like porous scaffolds using biodegradable polymers, injection of cells into the scaffolds to form cell/scaffold composites with a 3D structure, and implantation of these composites.

A scaffold is an artificially-made extracellular matrix (ECM) for constructing tissue and controlling cellular function. Accordingly, a scaffold requires biocompatibility in order not to cause transplant rejection, cellular toxicity, inflammatory reaction, and the like, as well as high efficiency to induce injection and proliferation of cells.

In particular, there has been demand for scaffolds having elasticity and flexibility for treatment suitable to a state of tissue reflecting individual features of defects, as the forms of bone defects vary depending on patients; that is, the size, shape, form, structure, *etc.* of the defects for treatment vary greatly.

Such suitable scaffolds are polylactic acid (PLA), polyglycolic acid (PGA), polycaprolactone (PCL), and copolymers thereof, which have been widely used for their relatively low toxicity, controllable degradability, and ease of preparation for tissue engineering scaffolds.

Meanwhile, electrospinning (ELSP), wherein the scaffold has porosity by the treatment with a biodegradable polymer solution, is a commonly used method for preparing scaffolds for tissue engineering (Guang-Zhen Jin *etc.*, Nanocomposite scaffolds incorporated with hydrophobically-functionalized mesoporous nanocarriers for the effective loading and longterm delivery of osteogenic drugs, RSC Adv., 2015, 5, 26832). However, electrospun nanofibers (ENs) fabricated by electrospinning formed as a very thin membrane, have rather small pores between nanofibers, which has been a limitation hindering cellular infiltration. Further, seeding cells only on the surface of the scaffold has been a limitation to availability depending on the size or shape of the bone defect. Such limitations have increased the demand for development of a scaffold which can be used in a free form and tuned to various bone defects according to patients' specific needs.

Patent US 2014/234457 A1 discloses an electrospinning device comprising: an electrospinning nozzle configured to discharge a polymer spinning solution by being applied with a high voltage in such a manner that the polymer spinning solution forms a fiber; a high voltage generator configured to apply a high voltage to the electrospinning nozzle; a ground power source configured to form an electric field in a space between the electrospinning nozzle and the ground power source so that the fiber discharged from the electrospinning nozzle is induced to flow in a predetermined direction by an electrostatic force; a gas spray nozzle configured to spray a gas in one direction; a collector configured to collect the fiber discharged from the electrospinning nozzle thereon, wherein the collector is disposed at a position opposed to that of the gas spray nozzle along the flow direction of the gas sprayed from the gas spray nozzle, and the fiber discharged from the electrospinning nozzle is collected on the collector by the flow force of the gas sprayed from the gas spray nozzle. discharged from the electro-spinning nozzle.

Patent KR20160073811A discloses a fibrous scaffold containing gelatin and PCL; And fiber scaffolds containing mesoporous bioactive glass nanospheres (mBGn). The scaffold is prepared by electrospinning.

With this background in mind, the present inventors have conducted studies to develop a scaffold which can be used in a form customized to various bone defects, and prepared scaffolds by electroblowing. As a result, the prepared scaffolds were confirmed to be shape-tunable cotton-like scaffolds with a 3D structure rather than a membrane form, thereby completing the present invention.

### [Disclosure]

### [Technical Problem]

An object of the present invention is to provide a method for preparing a cotton-like fibrous scaffold having a three-dimensional (3D) volume.

Another object of the present invention is to provide a cotton-like fibrous scaffold having a 3D volume prepared by the above method.

A further object of the present invention is to provide a stem cell/scaffold composite comprising the above fibrous scaffold.

### [Technical Solution]

In an aspect to achieve the objects, the present invention provides a method for preparing a fibrous scaffold comprising steps of:
a) forming an electric field by applying a voltage using a voltage generator between a nozzle of a syringe containing a biodegradable polymer solution and a current-collecting ground, wherein the biodegradable polymer is polycaprolactone; and
b) preparing a cotton-like fibrous scaffold having a three-dimensional (3D) volume by radially blowing the biodegradable polymer solution through the syringe nozzle under a continuous air flow by a fan.

A conventional scaffold for bone regeneration has been manufactured in the form of a thin membrane, in which the pores between nanofibers are dense, thereby hindering cellular infiltration. Further, the fact that seeding a cell is possible only on the surface of the scaffold has been a limitation in availability for various forms of defects, and specifically, the scaffold could not be used for a defect having a 3D shape. Under the circumstances, the present inventors confirmed that a scaffold prepared in a cotton-like form rather than a membrane form allows easy cellular infiltration because of the large pore between the fibers. Unlike a membrane-like scaffold, which is inapplicable to a huge defect site, a cotton-like scaffold was confirmed as a 3D platform enabling seeding cells into the scaffold, injecting the same in a 3D defect, and growing the bone vertically, thus completing the present invention.

Further, the preparation method of the present invention has an economic advantage in that the cotton-like form of the scaffold is prepared in the process for preparing the scaffold by enabling the fibrous network itself to be directly formed like cotton under a continuous air flow without requiring a separate secondary process to add physical force to a prepared scaffold.

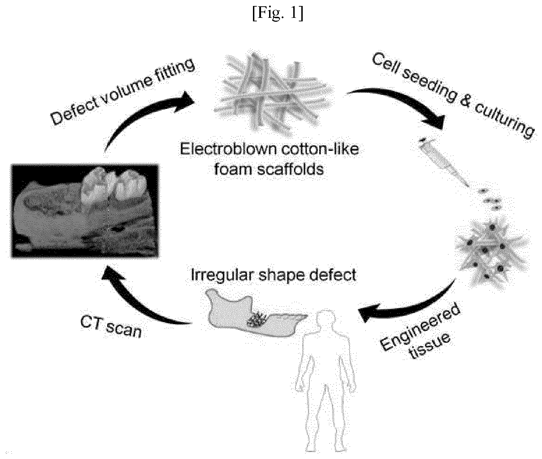

By seeding and culturing cells on the scaffold and preparing tissue required for implantation, the scaffold prepared by the method of the present invention can be used for the treatment personalized to patients having bone defects in various forms (Fig. 1).

The term "scaffold" in the present invention refers to a construct with which may partially substitute a defective organ or tissue *in vivo*; complement or substitute a function of the same; and specifically, treat a bone defect by regenerating bone tissue. The present invention prepared a scaffold particularly in a fibrous form to use for regenerating a bone.

The term "cotton-like" in the present invention is differentiated from a form of a two-dimensional thin film, and has properties of a 3D volume and an ability to flexibly change into various forms and sizes similarly to cotton if pressure is raised or lowered. Accordingly, the scaffold of the present invention can be injected in a form tuned to various shapes of bone defects.

Further, the cotton-like form is not prepared by swelling pores between fibers with physical force to a prepared scaffold, but is concurrently formed in the process for preparing the scaffold by blowing a biodegradable polymer solution under a continuous air flow.

Therefore, if a prepared scaffold is forced to be swollen by exerting secondary physical force, the sizes between pores become inconsistent, and the resulting form of the scaffold is difficult to predict. However, the cotton-like form of the present invention is formed as a network where the pore sizes are evenly increased under a continuous air flow and where fibers are evenly intertwined.

The term "biodegradable polymer" in step a) refers to a biocompatible polymer material forming a framework of the fibrous scaffold. The biodegradable polymer is

The voltage in step a) may be 10 kV to 30 kV; specifically, 15 kV to 25 kV. If the voltage is below 10 kV, the fiber may not be sufficiently prepared, and if the voltage is above 30 kV, a bead-like form may be prepared rather than a cotton-like form.

In the present invention, the speed of air flow in step b) may be 0.5 m/h to 5 m/h; specifically, 1 m/h to 2.5 m/h. If the speed of air flow is below 0.5 m/h, it may take too long to prepare a certain amount of fiber, and if the speed of air flow is above 5 m/h, a bead-like form may be prepared rather than a cotton-like form.

In the present invention, the cotton-like fibrous scaffold having a 3D volume, which is prepared in step (b), has larger pores among the fibers than those of a membrane-form fibrous scaffold, and accordingly is in a form easy to infiltrate a cell. Further, upon change of the pressure, the shape and size of the scaffold of the present invention can be flexibly changed, and the pore sizes thereof are even.

An exemplary embodiment of the present invention prepared a 3D cotton-like fibrous scaffold forming a fiber-intertwining network through steps of moving a polycaprolactone solution into a syringe and adding a voltage of 18 kV to 22 kV thereto under a continuous air flow of up to 2.5 m/h by an exterior fan (Example 1-2).

The biodegradable polymer solution may further comprise a bioactive glass nanoparticle (BGn). The "bioactive glass" refers to a material with superior bioactivity that enables chemical bonding with a tissue after implantation without accompanying toxicity, inflammation, and negative immune responses by forming a layer of hydroxyapatite, an ingredient similar to bone, on the surface.

The median diameter of the bioactive glass nanoparticle may be in a range of 1 nm to 999 nm; specifically, 1 nm to 300 nm; more specifically, 1 nm to 200 nm. An exemplary embodiment of the present invention confirmed that bioactive glass nanoparticles have a diameter of 42 ± 5 nm. Herein, a median diameter refers to a diameter corresponding to 50 wt% on a particle size distribution curve.

The bioactive glass nanoparticle may comprise 1 wt% to 20 wt% relative to the total weight of the biodegradable polymer solution; specifically, 5 wt% to 15 wt%; more specifically, 10 wt%; however, it is not limited thereto.

The bioactive glass nanoparticle may have a role in imparting an osteoinductive property to a cotton-like fibrous scaffold. In an exemplary embodiment of the present invention, scaffolds were prepared using a polycaprolactone (PCL) solution and a PCL solution containing 10% BGn. As a result, it was confirmed that the efficacy of cell proliferation and osteogenic differentiation were superior in the scaffold prepared using the PCL solution containing 10% BGn.

The bioactive glass nanoparticles may comprise silica (SiO₂) and calcium oxide (CaO) at a ratio of 60 to 80 : 20 to 40, based on mol%; specifically, 70 to 80 : 20 to 30; more specifically, 75:25; however, it is not limited thereto.

The bioactive glass nanoparticles may be prepared by a method comprising steps of
a) preparing a template solution by dissolving polyethylene glycol (PEG) using a sol-gel technique;
b) preparing a calcium oxide-template solution by adding a calcium oxide precursor to the template solution;
c) preparing a reaction product by stirring and ultrasonicating the calcium oxide-template solution while adding a silica precursor solution thereto; and
d) preparing mesoporous bioactive glass nanoparticles by centrifuging the reaction product followed by washing, drying, and sintering the resultants.

Further, the calcium oxide precursor in step b) may be selected from the group consisting of calcium nitrate tetrahydrate, calcium chloride, calcium acetate, and calcium methoxyethoxide, but is not limited thereto.

Additionally, the silica precursor of step c) may be selected from the group consisting of tetraethyl orthosilicate (TEOS), trimethoxy orthosilicate (TMOS), (3-glycidoxypropyl)methyldiethoxysilane (GPTMS), 3-mercaptopropyl trimethoxysilane (MPS), γ-glycidyloxypropyl trimethoxysilane (GOTMS), and aminophenyl trimethoxysilane (APTMOS), but is not limited thereto.

To achieve the objects, an exemplary embodiment of the present invention provides a cotton-like fibrous scaffold having a 3D volume. Specifically, the fibrous scaffold is a scaffold whose shape can be tuned to a defect site.

The scaffold of the present invention is prepared by evenly swelling the size of pores under a continuous air flow so that the entire thickness and volume of the scaffold are evenly increased. Further, as physical force is not exerted to expand the size of pores, there is no flaw or twist of the scaffold during the preparation, and a problem of the fibrous fine structure disintegrating does not occur.

In another aspect to achieve the objects, the present invention provides a stem cell/scaffold composite comprising the fibrous scaffold.

In the present invention, the stem cell can be directly involved in the regeneration of periodontal tissue by differentiation into an osteoblast, *etc.*, and can indirectly regenerate periodontal tissue by a certain factor secreted/expressed by a periodontal ligament cell. The stem cell/scaffold composite has biocompatibility upon implantation; has a remarkable effect of significantly increasing bone volume, bone surface, and bone density upon implantation into a periodontal defect model; and accordingly, can be used as a pharmaceutical composition for bone regeneration.

The scaffold of the present invention is easy to infiltrate a cell, and thereby enabling to seed the cell even inside the scaffold. In addition, the scaffold of the present invention is flexible to change its shape, and thereby enabling the bone regeneration customized to a shape of bone defect.

An exemplary embodiment of the present invention confirmed that when a stem cell/scaffold composite was injected into a rat model having a defect of periodontal ligament removal, there was an effect of significantly increased bone volume, bone surface, and bone density. In particular, when using a scaffold on which a stem cell was seeded, there was considerably improved formation of new alveolar bone (Example 4-3).

### [Advantageous Effects]

A scaffold prepared by the method of the present invention has a 3D cotton-like form and an ability to induce vertical regeneration of bone in a bone defect site, and can be used as a composition for bone regeneration being injected in a form personalized to patients having various bone defects.

### [Brief Description of Drawings]

Fig. 1 shows a process for preparing a tissue required for implantation by seeding and culturing cells on a scaffold as a method for treating defective bone in various forms personalized to patients using scaffolds and a tissue-engineered construct.
Fig. 2 shows characteristics of PCL EBC scaffolds. (a) EBC scaffold easily molded and compacted compared with electrospun membrane form. (b) EBC scaffold absorbing aqueous medium to maintain shape. (c) EBC scaffold taking up blood quickly (within minutes).
Fig. 3 shows EBC scaffolds prepared with the incorporation of 10% BGn to target bone regeneration. (a) SEM morphologies of pure PCL and 10% BGn-PCL scaffolds. (b) TEM image of 10% BGn-PCL scaffold having inserted EDS with 85Si/15Ca composition. (c) Ionic releases of silicate and calcium from EBC scaffold, measured by ICP-AES, for up to 28 days.
Fig. 4 shows cell anchorage (%) to an EBC scaffold surface measured at different time points; and significantly higher initial cell anchorage noticed in the 10% BGn-EBC scaffold than in the PCL scaffold.
Fig. 5 shows cell proliferation in the EBC scaffolds. (a) Cell viability measured for up to 9 days, as assayed by K method of confocal fluorescence images of cells in CC, and (b) (Phalloidin/DAPI stains) for each period.
Fig. 6 shows osteogenic differentiation of cells with long-term cultures of up to 3 weeks. (a) ALP activity measured at 3 weeks. (b) and (c) ARS stained optical images. (d) Quantification by eluting stains.
Fig. 7 shows *in vivo* bone formation ability of the EBC artificial scaffolds, examined by using a vertical bone augmentation model made in the calvarium defect using a cap. (a) Histological images of tissue samples at 6 weeks post-operation, by H&E stains at low and high magnification; original bone (OB) indicated by arrows; newly formed bone (NB) both in "calvarium" and "cap" regions discriminated by dotted lines. (b) Quantification of new bone area in "calvarium" and "cap" regions. (c) Osteocyte number counted in new bone area (calvarium + cap).
Fig. 8 shows bone formation analyzed by µ-CT. (a) 3D constructed and cross-sectional images of new bone formation (NB: calvarium; ^{∗}: cap region), and MIP color mapping images revealing quality of bone. (b) Bone formation index (bone volume, bone surface, and bone surface density) analyzed by µ-CT.

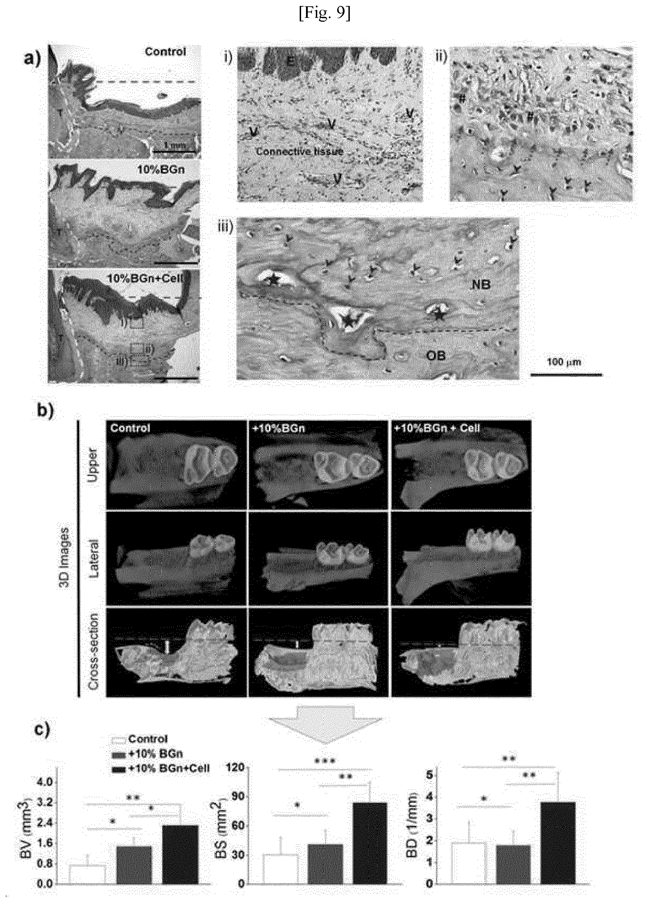

Fig. 9 shows capacity of the 10% BGn/rDPSC constructs to regenerate an irregular shaped defect in a rat alveolar bone. (a) H&E stained images of tissue samples at 8 weeks post-operation; epithelium (E), blood vessel (V), border of NB and OB (dotted line), bone forming front(arrows) with osteoblasts, bone lining cells, and osteoclasts mixed , osteocytes (arrowheads), and Harvesian canal (★). (b) µ-CT images of tissue samples represented in different modes; upper and lateral views and cross-sectional images with new bone highlighted. (c) µ-CT quantitative analyses of bone volume, bone surface, and surface density.

### [Detailed Description for Carrying Out Invention]

Hereinafter, the present disclosure will be described in detail with accompanying exemplary embodiments. The invention is as defined in the appended claims.

### Example 1: Preparation of mesoporous bioactive glass nanoparticles and cotton-like fibrous scaffold

The following materials for preparing bioactive glass nanoparticles and a scaffold were purchased from Sigma-Aldrich and were used as received without any further purification: tetraethyl orthosilicate (TEOS, C₈H₂₀O₄Si, 98%), calcium nitrate tetrahydrate (Ca(NO₃)₂·4H₂O, 99%), hexadecetyltrimethyl ammonium bromide (CTAB, C₁₉H₄₂BrN, ≥98%), ammonium hydroxide (NH₄OH, 28.0% NH₃ aqueous solution, ≥99.99% metal basis), methanol anhydrous (CH₃OH, 99.8%), poly(ε-caprolactone) (PCL, (C₆H₁₀O₂)ₙ, Mn = 80000), and chloroform (CHCl₃, 99.0%, Duksan chemicals, South Korea).

### 1-1. Preparation of mesoporous bioactive glass nanoparticles

Mesoporous bioactive glass nanoparticles were prepared by a sonochemical sol-gel technique utilizing CTAB as a template, as conventionally described.

In order to prepare bioactive glass nanoparticles, the constitution of 75% SiO₂/25% CaO (mol%) was used as follows. First, 13.72 mmol of CTAB and 1.27 mmol of Ca(NO₃)₂·4H₂O were solubilized in 120 mL of an alkaline methanol solution with pH adjusted to 12.5 using NH₄OH. Next, 3.79 mmol of TEOS was diluted with 30 mL of methanol and then added dropwise to the solution while vigorously stirring, then ultrasonicated for 20 min. After 24 h, the precipitate formed was separated, washed with water/ethanol, and then dried at 70°C overnight. The dried powder was heat-treated at 600°C for 5 h under a continuous air flow, and then stored under vacuum.

### 1-2. Preparation of cotton-like fibrous scaffold

Cotton-like fibrous scaffolds were fabricated using pure PCL and PCL added with 10 wt% BGn solutions.

Specifically, an electroblowing process was performed inside a metal framed plastic box (80 cm × 70 cm × 60 cm). A pure PCL (10% w/v) solution and a biodegradable polymer 10% BGn-PCL solution dissolved in chloroform were prepared and transferred to a plastic syringe (10 mL) with a metal nozzle (G-23). The syringe was equipped with a syringe pump, which was then adjusted at a feeding rate of 10 mL/h and kept at a distance of 35 cm from a stainless steel sheet mounted to the wall of the plastic box. The polymer solutions were electroblown at 18 kV to 22 kV under a continuous flow of a rotational air stream applied by an external fan (air speed up to 2.5 m/h) to immediately evaporate the solvent and to make 3D entangled fiber networks. The 3D fiber networks produced were collected manually by a plastic rod as a cotton-like foam.

### Experimental Example 1: Analysis of physicochemical properties of cotton-like fibrous scaffold

### 1-1. Observation of structure of cotton-like fibrous scaffold

The morphology of electroblown cotton-like foam (EBC) scaffolds fabricated in Example 1 above was observed by a high-resolution scanning electron microscope (SEM, JEOL, Japan). Prior to the SEM observation, the samples were coated with platinum for 90 seconds using an automatic magnetron sputter coater (Cressington 108 Auto sputter coater, UK). The morphology, mesostructure, and elemental composition of BGn were analyzed by a high-resolution transmission electron microscope (HR-TEM, JEM-3010, JEOL, Japan) equipped with an energy-dispersive X-ray spectrometer (EDX, Oxford Instruments, UK).

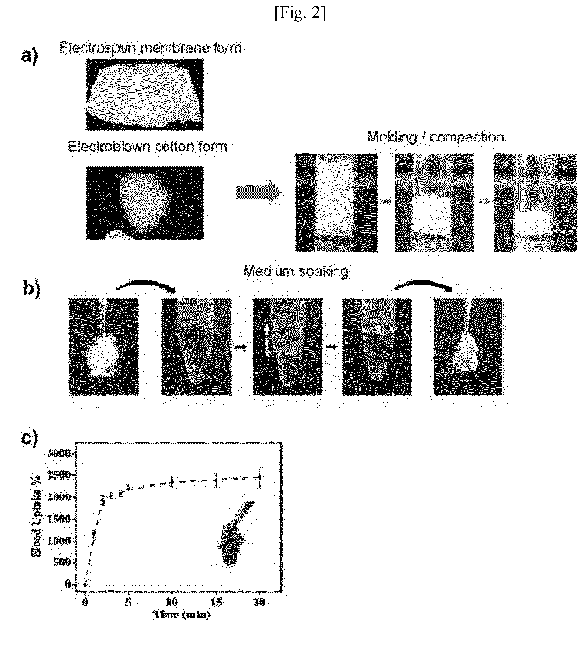

The EBC scaffold fabricated in Example 1 had a sponge form and very large pores among the fibers, in contrast to the small pores among the fibers of an electrospun membrane-form scaffold; and moreover, as presented in Fig. 2a, the EBC scaffolds could be easily molded and compacted to fit the shape and size of a mold compared with the membrane-form scaffold. Further, as shown in Fig. 2b, the EBC scaffold maintained its shape even after absorbing aqueous medium.

The blood uptake capacity of the EBC scaffold was measured by the blood immersion method using citrated rat blood. The weight of the EBC scaffold was measured before and after the blood immersion test at different time intervals to identify the degree of blood uptake.

As a result, the EBC scaffold quickly soaked within minutes, with a maximum blood uptake of 2300% (Fig. 2c).

### 1-2. Structural comparison by differences in EBC scaffold compositions

Differences in EBC scaffolds were observed by changing the solution composition. As BGn have been shown to be bone-bioactive and to stimulate differentiation of multipotent stem cells toward an osteogenic lineage, an EBC scaffold incorporating BGn as the bioactive nanocomponent was also prepared for bone regeneration.

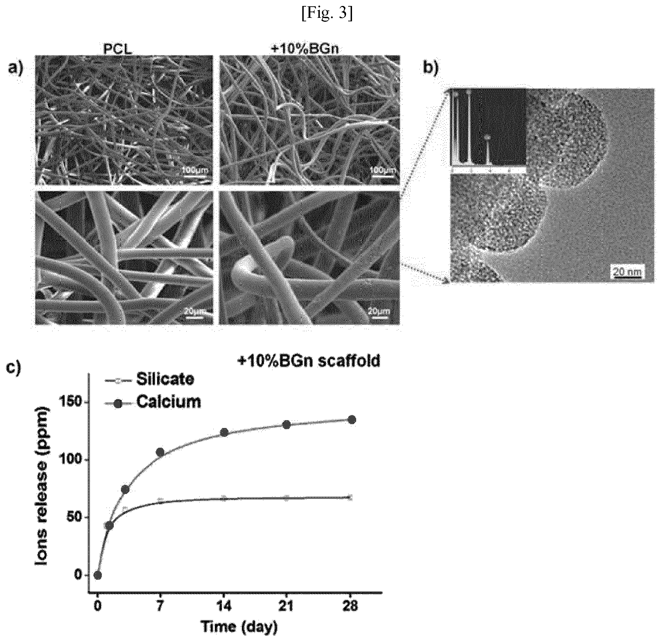

As presented in Fig. 3a, an EBC scaffold prepared with addition of 10% BGn to a PCL solution (10% BGn-EBC scaffold) was shown to generate a cotton-like fiber foam with a morphology similar to that of a scaffold prepared using a pure PCL solution. In addition, the average fiber size of 20.6 µm and 22.0 µm for the EBC and 10% BGn-EBC scaffolds, respectively, was in a similar range.

Meanwhile, as presented in Fig. 3b, the 10% BGn-EBC scaffold revealed a higher mesoporosity than the EBC scaffold.

### 1-3. Ion release

The cumulative release of silicate and calcium ions from EBC scaffolds containing 10% BGn was measured at 37°C with a shaking speed of 120 rpm. The EBC scaffold (100 mg) was immersed in a Tris-HCl buffer solution (15 mL) with pH adjusted to 7.4. At each predetermined time point, the release medium was collected and then refilled. The collected media was analyzed for ion release using an inductively coupled plasma atomic emission spectrometer (ICP-AES; OPTIMA 4300 DV, Perkin-Elmer, USA).

As shown in Fig. 3c, the 10% BGn-EBC scaffolds were also shown to release ions (silicate and calcium) as measured by ICP-AES. While the release of silicate ions appeared to saturate after 7 days, the calcium ions continued to release for up to 28 days.

The approximate ion concentrations released for 7 days were up to 60 ppm (up to 9 ppm/day) for silicate and up to 110 ppm (up to 16 ppm/day) for calcium, and these ionic levels are considered to be in a range in which 10% BGn-EBC scaffolds can exert therapeutic effects such as cell proliferation, angiogenesis, and osteogenesis.

### Experimental Example 2: In vitro effect in cell proliferation and osteogenic differentiation

### 2-1. Culture of dental pulp stem cells from rats

Rat dental pulp stem cells (rDPSCs) were extracted from the incisor teeth of 5-week-old male Sprague-Dawley (SD) rats. The extracted stem cells were digested in a solution of collagenase type I (Worthington Biochemical, USA) and dispase-II (neutral protease, USA) for 2 h at 37°C. Next, the collected cells were cultured in an α-minimal essential medium (MEM; HyClone, GELifesciences, USA) supplemented with 10% fetal bovine serum (FBS; HyClone, USA) and 1% penicillin/streptomycin (Gibco), and maintained at 37°C in a humidified atmosphere containing 5% CO₂. For differentiation into osteoblasts, the cells were cultured in an osteogenic medium (OM) containing ascorbic acid (50 µg/mL), 100 nM dexamethasone, and 10 mM β-glycerophosphate.

### 2-2. Cell adhesion to EBC scaffold

Sterilized EBC scaffolds (5 mg) were placed in each well of non-adherent 24-well plates (SPL Life Sciences, Korea). 50 µL of rDPSCs cells (1 × 10⁵) in normal media was seeded into each scaffold and then incubated at 37°C in a humidified atmosphere of 5% CO₂ for different time periods.

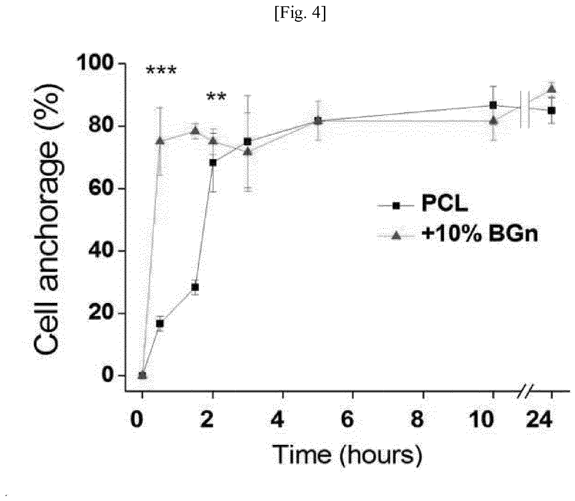

First, the cell adhesion behavior was examined with respect to culture time. An aliquot of cells (10⁵ cells in 50 µL) seeded quickly permeated through the scaffold (5 mg) to preserve the scaffold shape. As presented in Fig. 4, cells anchored to the 10% BGn-EBC scaffold very rapidly, with a population of up to 80% within 1 h, which then saturated within 24 h. However, on the EBC scaffold, the cell anchorage increased with time up to 3 h (up to 80%), more slowly than the 10% BGn-EBC scaffold, after which the cell population also saturated.

As a result, after sufficient time had passed, although the final populations of cells were comparable for both scaffolds, the initial anchorage behaviors were different. Through the observation, it was confirmed that cell anchorage largely depends on composition and culture period of a scaffold.

### 2-3. Cell proliferation on EBC scaffold

After 24 h of the cell adhesion stage, the DPSCs/scaffold constructs were cultured for longer periods in an osteogenic medium, and proliferation of DPSCs in the EBC scaffold was examined.

Cell viability was assessed using a cell counting assay (CCK-8, Dojindo Laboratories, Japan). The CCK-8 solution was added to the medium (at a 9:1 ratio) and then incubated at 37°C for 2 h. 100 µL of each reaction medium was transferred to 96-well plates, and absorbance was measured at 450 nm using a microplate reader (SpectraMax M2e, Molecular Devices, USA). Tests were performed in triplicate (n = 3).

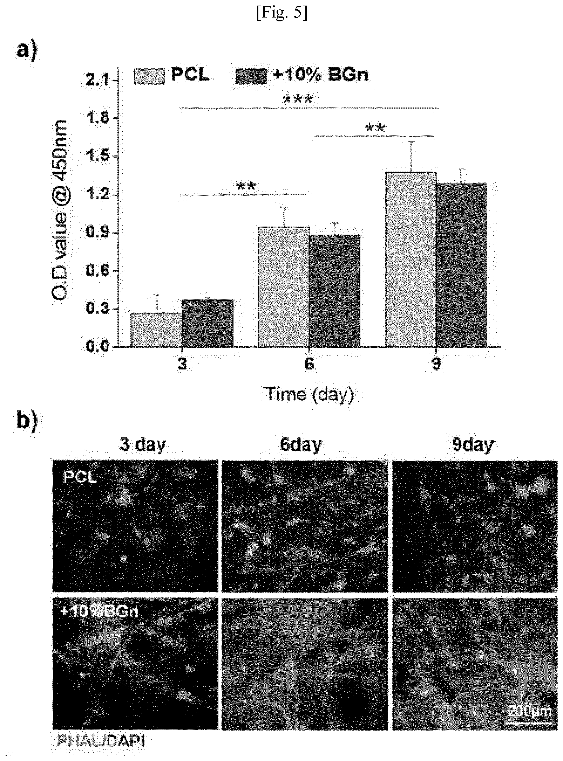

The CCK assay showed a continual increase in cell viability with time for up to 9 days for both scaffolds, with comparable levels (Fig. 5a).

Cell morphology was examined using a fluorescence microscope; the cells were fixed with 4% paraformaldehyde (PFA) solution at 4°C for 20 min and treated with 0.2% Triton X-100 (Sigma) for 5 min. After washing with PBS, the cells were stained with Alexa Flour 488 labeled (green) phalloidin (Life Technologies, Invitrogen) and then with DAPI (p36935, Invitrogen). Cell images were visualized under a confocal laser scanning microscope (CLSM; Zeiss LSM 700, Germany).

The CLSM fluorescence images of cells also supported the status of cells that were actively proliferating and highly viable over the culture period. Particularly, at day 9, a number of cells covered the scaffold surface almost entirely and exhibited highly extended cytoskeletal processes, bridging the fibers (Fig. 5b). The results confirmed that both scaffolds have excellent cell compatibility and the capacity to populate stem cells over a long period of time.

### 2-4. Osteogenic differentiation by ALP and ARS assays

To assess the osteogenic differentiation of rDPSCs grown on the scaffolds, the alkaline phosphatase (ALP) activity (Colorimetric kit, Abcam, UK) and Alizarin Red S (ARS; SIGMA-ALDRICH, USA) staining assays were introduced. Cells were seeded at 1 × 10⁵ into each scaffold, and cultured in an osteogenic medium for 7, 14, and 21 days.

For the ALP assay, the cells were collected using 0.25% trypsin and ETDA (Gibco). The ALP buffer (0.6 mL) was added to each sample, which was then centrifuged at 12000 rpm for 15 min at 4°C, and the supernatant was collected. The ALP activity (n = 5) was measured using *p*-nitrophenol phosphate (5 mM, 50 µL) after incubation for 60 min at 25°C. The absorbance was read at a wavelength of 405 nm using a microplate reader, and data was normalized to the double-stranded DNA (dsDNA) quantity measured by the dsDNA detection kit (Quant-iT Picogreen, Invitrogen) following standard protocols.

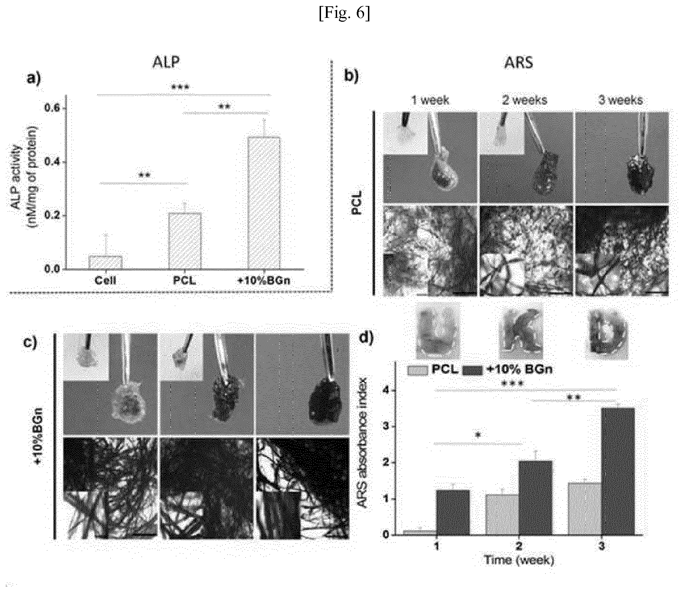

The osteogenic differentiation of DPSCs upon the EBC scaffolds was investigated by analyzing the ALP activity of cells during culture for 3 weeks. As presented in Fig. 6a, compared to the cells cultured on a tissue culture dish, those cultured on the EBC scaffolds presented significantly higher ALP activity levels. Between the two scaffold groups, 10% BGn-EBC scaffolds were more effective than EBC scaffolds in stimulating cellular ALP activity.

The cell mineralization behaviors were then examined by ARS assay for 3 weeks. For the ARS staining, the cell-seeded scaffolds were stained with a 40 mM ARS assay kit (pH 4.2) for 10 min under gentle rotation. The staining level was visualized under a light microscope (IX71, Olympus, Tokyo, Japan). For quantification, 10% cetylpyridinium chloride (Sigma) in sodium phosphate (pH 7.0 and 10 mM) was added to elute the stains, and then absorbance was measured at 562 nm using a microplate reader.

As presented in Figs. 6b and 6c, the ARS stained optical images showed a substantial increase in stain density with culture time, confirming that the mineralization occurred significantly over the culture period. The stain intensities were homogeneous throughout the scaffolds. Images at higher magnification revealed the distribution of mineralized nodules along the fibers. Moreover, the stains in the 10% BGn-EBC scaffold were more evident compared with the EBC PCL scaffold.

When the mineralization was quantified by eluting the stains with 10% CPC solution, there was significant difference between the groups, as presented in Fig. 6d (control group < PCL < 10% BGn).

Inset are the optical images of cell/scaffold constructs made in "U", "K", and "D" molds at 1, 2, and 3 weeks, respectively, post ARS staining, representing the possibility of use when tuning the shape to defects in various forms by using the EBC scaffolds.

### Experimental Example 3: In vivo efficacy in capped calvarium defect model of rats

Through Experimental Example 2 above, it was confirmed that the EBC scaffolds prepared in Example 1 could provide excellent scaffolding conditions for stem cells to populate, differentiate into an osteogenic lineage, and then mature to mineralize, and the *in vivo* performance for bone regeneration of the EBC scaffolds was examined in rats.

### 3-1. Implantation into capped calvarium defect of rats

12-week-old, 180 g to 220 g healthy male Sprague-Dawley rats were used in the surgery. The animals were randomly allocated to one of the following three groups before implantation of scaffolds: control, EBC scaffold, and 10% BGn-EBC scaffold groups.

The assessment of the capacity of EBC scaffolds to induce bone regeneration ability was conducted with a capped calvarium defect model of rat. After shaving over the cranial lesion, the surgical site was scrubbed with iodine and 70% ethanol, and a linear skin incision was made. A full-thickness flap was retracted and the calvarial bone was exposed. Two 5 mm diameter partial-thickness calvarial bone defects were prepared in each rat on each side of the parietal bone under cooling conditions with sterile saline using a dental handpiece and a 5 mm diameter LS-Reamer (Neobiotech, Seoul, South Korea).

Implant placement was performed under general anesthesia using an intramuscular injection of a mixture of ketamine (80 mg/kg) and xylazine (10 mg/kg). Rigid polymer caps (5 mm inner diameter × 2 mm height) were custom-made (Taulman 618 Nylon, Taulman 3D, Missouri, US) by 3D printing (NP-Mendel, Opencreators, South Korea). The EBC scaffolds were fixed with 3D-printed rigid polymer caps where the cap and scaffolds were sheltered to the calvarial bone using fixation screws via its anchoring rings. The subcutaneous tissues and periosteum were sutured with absorbable sutures (4-0 Vicryl^{®}, Ethicon, Germany), and the skin was closed with non-absorbable suture material (4-0 Prolene, Ethicon, Germany).

After the implantation of the scaffolds, the animals were monitored daily for possible clinical signs of infection, inflammation, and any adverse reaction. After six weeks, the animals were euthanized by CO₂ inhalation, and the tissue part of the calvarium surrounding the cap was harvested and fixed in 10% neutral buffered formalin for 24 h at room temperature.

### 3-2. Histological imaging

Six weeks post implantation, the tissue samples were harvested and the histological images were analyzed optically after H&E staining.

For the histological analysis, the cap was removed after tissue fixation, and the fixed tissues were subsequently decalcified in RapidCal^{™} solution (BBC Chemical Co., USA) for 3 days. After decalcification, the samples were dehydrated in ascending grades of ethanol and then embedded in paraffin. 5 µm coronal sections of the central area at the defect site were prepared using a semi-automated rotary microtome (Leica RM2245, Leica Biosystems, Germany) and were then moved to coated glass slides. The slides with tissue sections were de-paraffinized and hydrated through a series of xylene and ethanol solutions, and were finally stained with hematoxylin and eosin (H&E) for the assessment of new bone area. Histological sections were visualized under a light microscope (IX71, Olympus, Japan).

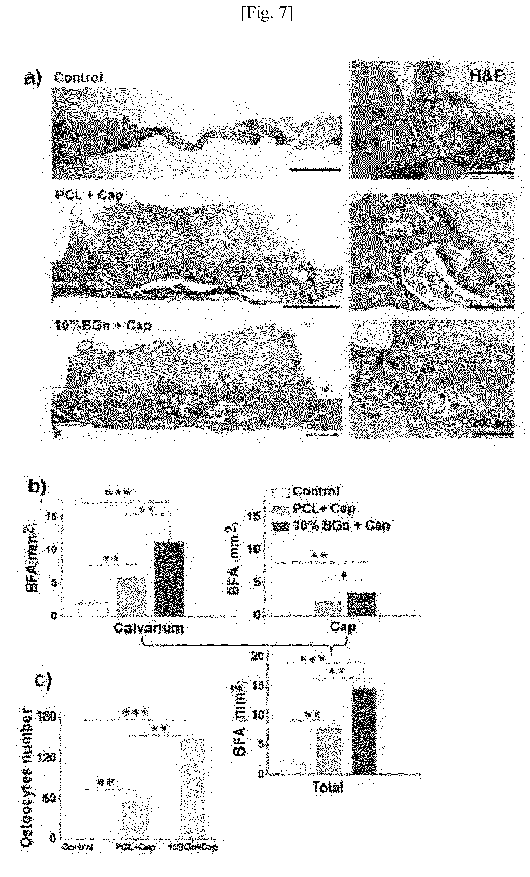

As presented in Fig. 7a, in the control group, the new bone formation in the calvarium region was only minimal. However, when the EBC scaffold was implanted, new bone formation was observed in the calvarium region, while the cap region was filled mostly with soft tissues. Bone-like tissues were mainly observed along the cap surface, suggesting that the vertical bone growth was more likely from the edges of the defect. On the other hand, when the 10% BGn-EBC scaffold was implanted, the new bone formation in the calvarium region was more significant, and the vertical bone growth in the cap region was also noticeable even at the central zone.

As revealed in the histology, the new bone formation in the EBC scaffold was mostly along the cap wall surface, whereas the new bone growth was quite homogeneous vertically (not only along the cap wall but also through the central part) in the 10% BGn-EBC scaffold. This implies that the 10% BGn-EBC scaffold has greater osteoinductive capacity for vertical bone growth than the EBC scaffold.

The new bone area in the calvarium and cap regions was then quantified as presented in Fig. 7b. Both scaffolds were significantly effective in enhancing the new bone formation in calvarium region, with a higher statistical significance analyzed in the 10% BGn-EBC scaffold. The new bone formation in the cap region was also significantly enhanced by the scaffolds, and in particular, the significance was greater in the 10% BGn-EBC scaffold.

The osteocyte number counted in the new bone area (calvarium + cap) was also significantly enhanced in the scaffold groups, and the level observed in the 10% BGn-EBC scaffold was about 2.5 times higher (Fig. 7c).

### 3-3. Micro imaging

The bone formation was further analyzed by µ-CT. Micro-computed tomography (µ-CT) imaging was conducted (Skyscan 1176, Skyscan, Aartselaar, Belgium) by X-ray at 65 kV and 385 µA, with an exposure time of 279 ms for each section (µm). Reconstructed images from scanned images were made into 3D images visualized by CTvol Skyscan software (ver. 2.3.2.0), and were used to analyze hard tissue formation over the region of interest. Specifically, new hard tissue volume (%), hard tissue surface (mm2), and hard tissue surface density (1/mm) in the defect area were analyzed.

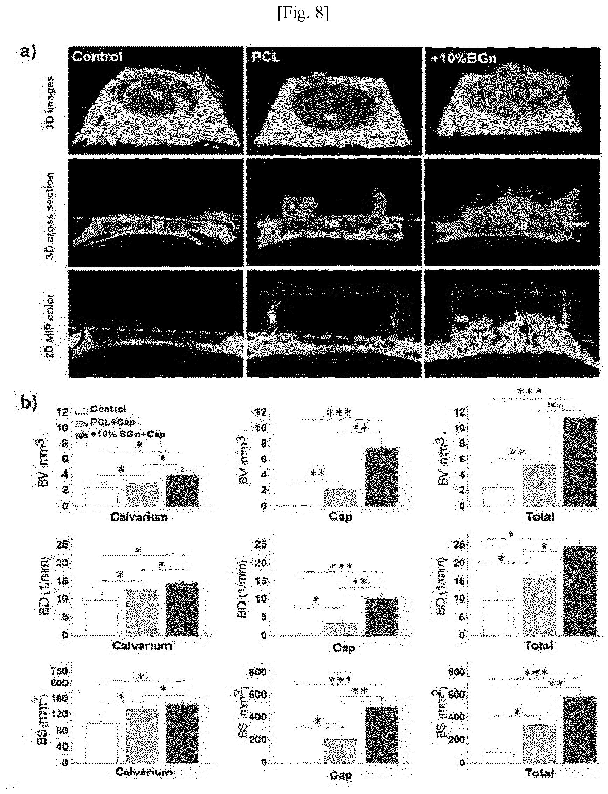

The 3D constructed and cross-sectional images (NB: calvarium; ^{∗}: cap region) and the MIP color mapping images revealed the quantity and quality of newly formed bone (Fig. 8a). Based on the images, bone formation indices, including bone volume, bone surface, and bone surface density, were calculated.

As presented in Fig. 8b, the new bone volume in the calvarium region was in the order of empty control < PCL < +10% BGn. In the cap region, the new bone was only formed in the scaffold groups, with an especially higher level in the 10% BGn-EBC scaffold. The new bone area and surface density also showed a similar trend to the bone volume. The µ-CT results coincided with the histological results. It was confirmed that the EBC scaffold offers an excellent 3D scaffolding condition for bone ingrowth, and that the BGn component in the scaffold stimulates vertical bone growth.

### Experimental Example 4: In vivo efficacy in tooth extract mandible defect model of rats

On the basis of the excellent bone forming ability of 10% BGn-EBC scaffolds, the potential of the cell-seeded scaffolds for the regeneration of bone was examined.

### 4-1. Implantation into tooth extract mandible defect model

Upper first molar teeth were extracted from each side of the maxilla socket of rats. A dental socket was made as an artificial defect using a round shaped dental burr (φ = 1.6 mm) under cooling conditions with sterile saline using a dental high-speed handpiece (Sirona, Germany) until bifurcation bone was removed (Fig. 7a). The scaffolds were filled to a first upper maxilla socket hall, and sheltered to the alveolar bone using a non-absorbable suture material (4-0 Prolene, Ethicon, Germany). The DPSCs were cultivated in the 10% BGn-EBC scaffold for 7 days in an osteogenic medium, which was used for the implantation in the tooth-extracted alveolar bone defect.

### 4-2. Histological imaging

The tissue samples were histologically examined after H*&*E staining. The borders of the new bone area are indicated by arrowheads (NB: new bone; OB: old bone).

From the µ-CT results, compared with the control where only minimal quantity of bone was formed, as presented in Fig. 9a, the 10% BGn-EBC and 10% BGn/rDPSC EBC scaffolds showed substantial bone formation.

A higher-magnification image (iii) of the 10%BGn/rDPSC group revealed a highly matured structure of the new bone area (NB), which was similar to that of the old bone area (OB). A number of osteocytes (arrowheads) were present in lacunae, and the Harvesian canal (★) with lamella patterns was developed.

The enlarged image of a middle part (ii) presenting the front of a bone forming zone (arrows) revealed a number of osteoblasts and bone lining cells, and some osteoclasts. The upper part (i), filled with connective tissues, revealed blood vessels (V) below epithelium (E; gingival mucosa).

The histological images demonstrated the dynamic process of a new bone formation. Clearly, the rDPSCs introduced with EBC scaffolds were shown to play an important role in stimulating new vascularization and new bone formation in the mandible tooth socket defect, significantly increasing the bone volume and density.

### 4-3. Micro imaging

The µ-CT images of the tissue samples were achieved at 8 weeks post operation and presented as upper, lateral and cross-sectional views.

As presented in Fig. 9b, in the control group, the defect became somewhat collapsed (*i.e*., bone height lowered, as indicated by the curved green line) due to the lack of support by the tooth loss, thus requiring alveolar bone augmentation to preserve the bone height. When the EBC or 10% BGn/rDPSC EBC scaffold was used, the height of the gums was well preserved, demonstrating a merited aspect of the scaffolds for clinical applications.

The quantitative analyses of µ-CT images provided new bone volume, bone surface, and surface density. As presented in Fig. 9c, the bone volume and surface area were significantly higher in the group implanted with the 10% BGn-EBC and 10% BGn/rDPSC EBC scaffolds compared to the control group. Furthermore, based on the µ-CT analyses, the seeding of rDPSC cells into scaffolds showed more significant effects on new alveolar bone formation.

## Claims

1. A method for preparing a fibrous scaffold comprising steps of:
a) forming an electric field by applying a voltage using a voltage generator between a nozzle of a syringe containing a biodegradable polymer solution and a current-collecting ground, wherein the biodegradable polymer is polycaprolactone; and
b) preparing a cotton-like fibrous scaffold having a three-dimensional (3D) volume by radially blowing the biodegradable polymer solution through the syringe nozzle under a continuous air flow by a fan.

2. The method of claim 1 , wherein the voltage in step a) is 10 kV to 30 kV.

3. The method of claims1 or 2, wherein a speed of the air flow in step b) is 1 m/h to 2.5 m/h.

4. The method of any one of claims 1 to 3, wherein the biodegradable polymer solution in step a) further comprises bioactive glass nanoparticles.

5. The method of claim 4, wherein the bioactive glass nanoparticles are contained in an amount of 1 wt% to 20 wt% relative to the total weight of the biodegradable polymer solution.

6. The method of claim 4 or 5, wherein the bioactive glass nanoparticles comprise silica (SiO₂) and calcium oxide (CaO) at a ratio of 60 to 80 : 20 to 40, based on mol%.

7. The method of any one of claims 4 to 6, wherein the bioactive glass nanoparticles are prepared by a method comprising steps of:
a) preparing a template solution by dissolving polyethylene glycol (PEG) using a sol-gel technique;
b) preparing a calcium oxide-template solution by adding a calcium oxide precursor to the template solution;
c) preparing a reaction product by stirring and ultrasonicating the calcium oxide-template solution while adding a silica precursor solution thereto; and
d) preparing mesoporous bioactive glass nanoparticles by centrifuging the reaction product followed by washing, drying, and sintering the resultants.

8. The method of claim 7, wherein the calcium oxide precursor of step b) is selected from the group consisting of calcium nitrate tetrahydrate, calcium chloride, calcium acetate, and calcium methoxyethoxide.

9. The method of claim 7 or 8, wherein the silica precursor of step c) is selected from the group consisting of tetraethyl orthosilicate (TEOS), trimethoxy orthosilicate (TMOS), (3-glycidoxypropyl)methyldiethoxysilane (GPTMS), 3-mercaptopropyl trimethoxysilane (MPS), γ-glycidyloxypropyl trimethoxysilane (GOTMS), and aminophenyl trimethoxysilane (APTMOS).

10. A cotton-like fibrous scaffold having a 3D volume prepared by any one method of claims 1 to 9.

11. A fibrous scaffold of claim 10, wherein the fibrous scaffold has shape-tunability to a defect site.

12. A stem cell/scaffold composite comprising the fibrous scaffold of claim 10.

## Patentansprüche

1. Verfahren zur Herstellung eines Fasergerüsts, das die folgenden Schritte umfasst:
a) Bilden eines elektrischen Felds durch Anlegen einer Spannung unter Verwendung eines Spannungsgenerators zwischen einer Düse einer Spritze, die eine biologisch abbaubare Polymerlösung enthält, und einer Strom sammelnden Masse, wobei das biologisch abbaubare Polymer Polycaprolacton ist; und
b) Herstellen eines baumwollartigen Fasergerüsts, das ein dreidimensionales (3D) Volumen aufweist, durch radiales Blasen der biologisch abbaubaren Polymerlösung durch die Spritzendüse unter einer ununterbrochenen Luftströmung durch ein Gebläse.

2. Verfahren nach Anspruch 1, wobei die Spannung in Schritt a) 10 kV bis 30 kV beträgt.

3. Verfahren nach Anspruch 1 oder 2, wobei eine Geschwindigkeit der Luftströmung im Schritt b) 1 m/h bis 2,5 m/h beträgt.

4. Verfahren nach einem der Ansprüche 1 bis 3, wobei die biologisch abbaubare Polymerlösung im Schritt a) ferner bioaktive Glasnanopartikel umfasst.

5. Verfahren nach Anspruch 4, wobei die bioaktiven Glasnanopartikel in einer Menge von 1 Gew.-% bis 20 Gew.-%, bezogen auf das Gesamtgewicht der biologisch abbaubaren Polymerlösung, enthalten sind.

6. Verfahren nach Anspruch 4 oder 5, wobei die bioaktiven Glasnanopartikel Siliziumdioxid (SiO₂) und Kalziumoxid (CaO) in einem Verhältnis von 60 bis 80 : 20 bis 40, basierend auf Mol-%, umfassen.

7. Verfahren nach einem der Ansprüche 4 bis 6, wobei die bioaktiven Glasnanopartikel durch ein Verfahren hergestellt werden, das die folgenden Schritte umfasst:
a) Herstellen einer Templat-Lösung durch Auflösen von Polyethylenglykol (PEG) unter Verwendung einer Sol-Gel-Technik;
b) Herstellen einer Kalziumoxid-Templat-Lösung durch Beimengen eines Kalziumoxidvorläufers zu der Templat-Lösung;
c) Herstellen eines Reaktionsprodukts durch Rühren und Ultraschallbehandlung der Kalziumoxid-Templat-Lösung bei gleichzeitigem Beimengen einer Siliziumdioxid-Vorläufer-Lösung dazu; und
d) Herstellen von mesoporösen bioaktiven Glasnanopartikeln durch Zentrifugieren des Reaktionsprodukts, gefolgt von Waschen, Trocknen und Sintern der Ergebnisse.

8. Verfahren nach Anspruch 7, wobei der Kalziumoxidvorläufer von Schritt b) aus der Gruppe ausgewählt wird, die besteht aus Kalziumnitrat-Tetrahydrat, Kalziumchlorid, Kalziumacetat und Kalzium-Methoxy-Ethoxid.

9. Verfahren nach Anspruch 7 oder 8, wobei der Siliziumoxidvorläufer von Schritt c) ausgewählt wird aus der Gruppe, die besteht aus Tetraethylorthosilicat (TEOS), Trimethoxyorthosilikat (TMOS), (3-Glycidoxypropyl)methyldiethoxysilan (GPTMS), 3-Mercaptopropyl-Trimethoxysilan (MPS), γ-Glycidyloxypropyl-Trimethoxysilan (GOTMS) und Aminophenyl-Trimethoxysilan (APTMOS)

10. Baumwollartiges Fasergerüst mit einem 3D-Volumen, das durch ein Verfahren nach Anspruch 1 bis 9 hergestellt wurde.

11. Fasergerüst nach Anspruch 10, wobei das Fasergerüst eine Formabstimmbarkeit auf eine Defektstelle aufweist.

12. Stammzellen/Gerüst-Verbundmaterial, welches das Fasergerüst nach Anspruch 10 umfasst.

## Revendications

1. Procédé pour préparer une structure fibreuse, comprenant les étapes de :
a) formation d'un champ électrique par application d'une tension utilisant un générateur de tension entre l'embout d'une seringue contenant une solution de polymère biodégradable et une masse collectrice de courant, dans laquelle le polymère biodégradable est la polycaprolactone ; et
b) préparer une structure fibreuse analogue à du coton ayant un volume tridimensionnel (3D) par soufflage radial de la solution de polymère biodégradable à travers l'embout de seringue sous un courant d'air continu au moyen d'un ventilateur.

2. Procédé selon la revendication 1, dans lequel la tension dans l'étape a) est de 10 kV à 30 kV.

3. Procédé selon la revendication 1 ou 2, dans lequel la vitesse du courant d'air dans l'étape b) est de 1 m/h à 2,5 m/h.

4. Procédé selon l'une quelconque des revendications 1 à 3, dans lequel la solution de polymère biodégradable dans l'étape a) comprend en outre des nanoparticules de verre bioactif.

5. Procédé selon la revendication 4, dans lequel les nanoparticules de verre bioactif sont contenues en une quantité de 1 % en poids à 20 % en poids par rapport au poids total de la solution de polymère biodégradable.

6. Procédé selon la revendication 4 ou 5, dans lequel les nanoparticules de verre bioactif comprennent de la silice (SiO₂) et de l'oxyde de calcium (CaO) en un rapport, basé sur les pourcentages en moles, de 60/80 à 20/40.

7. Procédé selon l'une quelconque des revendications 4 à 6, dans lequel les nanoparticules de verre bioactif sont préparées par un procédé comprenant les étapes de :
a) préparation d'une solution de matrice par dissolution de polyéthylèneglycol (PEG) utilisant une technique sol-gel ;
b) préparation d'une solution de matrice d'oxyde de calcium par addition d'un précurseur d'oxyde de calcium à la solution de matrice ;
c) préparation d'un produit réactionnel par agitation et ultrasonification de la solution de matrice d'oxyde de calcium cependant qu'une solution de précurseur de silice y est ajoutée ; et
d) préparation de nanoparticules de verre bioactif mésoporeux par centrifugation du produit réactionnel suivie d'un lavage, d'un séchage, et d'un frittage des produits résultants.

8. Procédé selon la revendication 7, dans lequel le précurseur d'oxyde de calcium de l'étape b) est choisi dans le groupe constitué par le nitrate de calcium tétrahydraté, le chlorure de calcium, l'acétate de calcium, et le méthoxyéthylate de calcium.

9. Procédé selon la revendication 7 ou 8, dans lequel le précurseur de silice de l'étape c) est choisi dans le groupe constitué par l'orthosilicate de tétraéthyle (TEOS), le triméthoxyorthosilicate (TMOS), le (3-glycidoxypropyl)méthyldiéthoxysilane (GPTMS), le 3-mercaptopropyltriméthoxysilane (MPS), le γ-glycidyloxypropyltriméthoxysilane (GOTMS), et l'aminophényltriméthoxysilane (APTMOS).

10. Structure fibreuse analogue à du coton ayant un volume 3D préparé par un procédé selon l'une quelconque des revendications 1 à 9.

11. Structure fibreuse selon la revendication 10, laquelle structure fibreuse a une capacité d'adaptation de forme à un site de défaut.

12. Composite de cellules souches/structure comprenant la structure fibreuse selon la revendication 10.
